# EUROPEAN PATENT APPLICATION

(11) **EP 2 554 201 A1**
(43) Date of publication of application: **06.02.2013**
(21) Application number: 11765462.4
(22) Date of filing: 25.03.2011
(51) Int. Cl.: A61M 5/24, A61M 5/28, A61M 5/32

(54) **PREFILLED SYRINGE AND METHOD FOR ASSEMBLING PREFILLED SYRINGE**

(30) Priority: 31.03.2010 JP 2010082579
(71) Applicant: Terumo Kabushiki Kaisha, Tokyo 151-0072 (JP)
(72) Inventor: OKAYAMA, Tomoki, Nakakoma-gun Yamanashi 409-3853 (JP); TACHIKAWA, Kouichi, Nakakoma-gun Yamanashi 409-3853 (JP); OGAWA, Junichi, Nakakoma-gun Yamanashi 409-3853 (JP)
(74) Representative: TBK
(86) International application number: PCT/JP2011/057467
(87) International publication number: WO 2011/125561

(57) **Abstract**

To make it possible to keep a second needle tip of a needle tube in a sterilized state until it punctures a medicinal solution storage section in which a medicinal solution is filled. A prefilled syringe 1 includes a double-ended needle 3, an outer tube 2, a needle hub 4, a medicinal solution storage section 6 in which a medicinal solution M is filled, a pusher 5, and a cover member 17. The double-ended needle 3 has a first needle tip 9 provided at one end thereof which is to puncture a living organism, and has a second needle tip 10 provided at the other end thereof through which medicinal solution M is injected. The needle hub 4 holds an intermediate portion of the double-ended needle 3 and is disposed so as to close up the opening on one end side of the outer tube 2. The movable pusher 5 operates the medicinal solution storage section 6 for movement in an axial direction of the outer tube 2. The cover member 17 covers the second needle tip 10 side of the double-ended needle 3 exposed from the needle hub 4.

## Description

### TECHNICAL FIELD

The present invention relates to a prefilled syringe wherein a medicinal solution is accommodated in a syringe in advance and an assembling method of the prefilled syringe.

### BACKGROUND ART

In recent years, a prefilled syringe wherein a medicinal solution is filled in a syringe in advance has been and is used progressively (Patent Document 1). Such a prefilled syringe as just described eliminates the necessity to suck a medicinal solution into a syringe from a vial container upon medicinal administration and can reduce the time required for the administration and besides can reduce wasted the medicinal solution.

Also a technology has been proposed wherein, in external air to prevent a medicinal solution from touching the external air through a needle tube to keep the medicinal solution in a sterilized state and in stability, a medicinal solution is filled into a sealed vessel such as a medicinal solution storage section in advance and then the medicinal solution storage section in which the medicinal solution is filled is inserted into a cylindrical hole of an outer tube which is a syringe. Further, in the case of a prefilled syringe wherein a medicinal solution is filled in the medicinal solution storage section, a needle tube of a double-ended structure having a first needle tip for puncturing a living organism and a second needle tip for puncturing the medicinal solution storage section is used.

It is to be noted that, in order to administrate the medicinal solution, the medicinal solution storage section in which the medicinal solution is filled is pushed out in an axial direction of the outer tube by a pusher to cause the second needle tip to puncture the medicinal solution storage section. Then, the medicinal solution storage section is further pushed by the pusher to administrate the medicinal solution into the living organism.

### Prior Art Document

### [Patent Documents]

Patent Document 1: Japanese Patent Laid-Open No. 2004-321826

### SUMMARY OF INVENTION

### Problem to be Solved by the Invention

However, in the case of a prefilled syringe of the medicinal solution storage section type, even if the medicinal solution is filled in the medicinal solution storage section in order to keep the medicinal solution in a sterilized state, the second needle tip to puncture the medicinal solution storage section and touch with the medicinal solution is exposed to the external air in the cylindrical hole of the outer tube. Therefore, in order to further improve the sterilized state of the medicinal solution, it is demanded to keep the second needle tip, which is to touch the medicinal solution, in a sterilized state until it punctures the medicinal solution storage section.

In view of above problem, the object of the present invention is to provide a prefilled syringe wherein a second needle tip of a needle tube can be kept in a sterilized state until it punctures a medicinal solution storage section and touches a medicinal solution and an assembling method of the prefilled syringe.

### Means for Solving Problem

In order to solve the subject described above and achieve the object of the present invention, a prefilled syringe of the present invention includes a double-ended needle having a first needle tip provided at one end thereof for puncturing a living organism and a second needle tip provided at the other end thereof for being injected with a medicinal solution. The prefilled syringe further includes an outer tube having a cylindrical hole into which the second needle tip of the double-ended needle is disposed and which is open at the opposite ends thereof, a needle hub configured to hold an intermediate portion of the double-ended needle and close up the opening on the one end side of the outer tube, and a medicinal solution storage section movably inserted in the outer tube and filled with the medicinal solution by a sealing member which seals the one end. The prefilled syringe further includes a movable pusher configured to operate the medicinal solution storage section for movement in an axial direction of the outer tube, and a cover member configured to cover the second needle tip side of the double-ended needle exposed from the needle hub.

Meanwhile, an assembling method of a prefilled syringe according to the present invention includes steps indicated by (1) to (3) given below:
(1) a step of fixing a needle tube, which has a first needle tip provided at one end thereof and configured to puncture a living organism and a second needle tip having a medicinal solution injected therein and disposed in a cylindrical hole of an outer tube, to a needle hub and covering the second needle tip side of the needle tube held by the needle hub with a cover member,
(2) a step of applying a sterilization process to the needle tube, whose second needle tip is covered with the cover member, and the needle hub, and
(3) a step of inserting, into the cylindrical hole of the outer tube whose opening on the one end side is closed up with the needle hub, the medicinal solution storage section in which the medicinal solution is filled and a pusher provided so as to operate the medicinal solution storage section for movement in an axial direction of the cylindrical hole from the other end side of the outer tube under a sterilized environment.

### Effect of the Invention

With the prefilled syringe of the present invention, since the second needle tip which is to puncture the medicinal solution storage section is covered with the cover member, the second needle tip can be maintained in a sterilized state until it punctures the medicinal solution storage section and is brought into contact with the medicinal solution.

With the assembling method of the prefilled syringe of the present invention, since a sterilization process is carried out with the second needle tip side of the needle tube covered with the cover member, the sterilized state of the first needle tip and the second needle tip of the needle tube can be maintained. As a result, also upon assembly, the sterilized state of the second needle tip can be further maintained.

### BRIEF DESCRIPTION OF DRAWINGS

FIG. 1 is a sectional view showing a first embodiment of a prefilled syringe of the present invention.
FIG. 2 is a sectional view illustrating a state during administration of medicine in the first embodiment of the prefilled syringe of the present invention.
FIG. 3 is a sectional view illustrating a state in which the administration of medicine in the first embodiment of the prefilled syringe of the present invention is completed.
FIG. 4 is a sectional view showing a second embodiment of the prefilled syringe of the present invention.
FIG. 5 is a sectional view illustrating a state during administration of medicine in the second embodiment of the prefilled syringe of the present invention.
FIG. 6 is a sectional view illustrating a state in which the administration of medicine in the second embodiment of the prefilled syringe of the present invention is completed.

### MODE FOR CARRYING OUT THE INVENTION

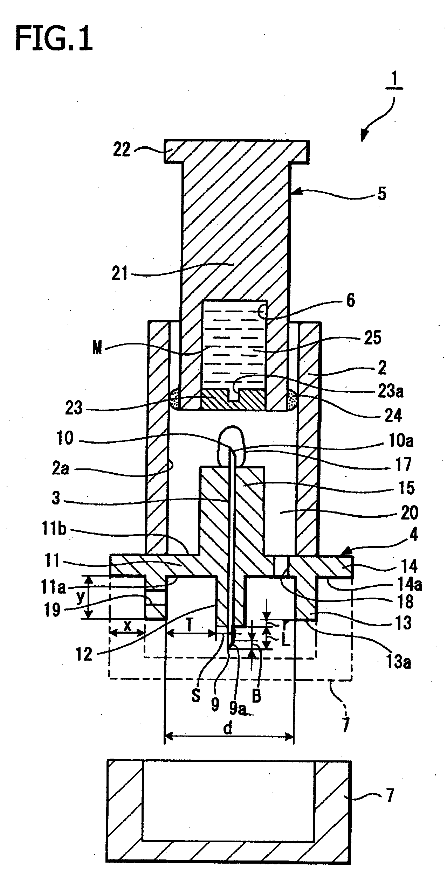

In the following, embodiments of the prefilled syringe of the present invention are described with reference to FIGS. 1 to 6. It is to be noted that, in the figures, like members are denoted by like reference letters or numerals. Further, the present invention is not limited to the following embodiments. Incidentally, description will be made in the following order.
1. First Embodiment
   1-1. Example of the Configuration of the Prefilled Syringe
   1-2. Assembling Method of the Prefilled Syringe
   1-3. Method of Use of the Prefilled Syringe
2. Second Embodiment

### <1. First Embodiment

### 1-1. Example of the Configuration of the Prefilled Syringe

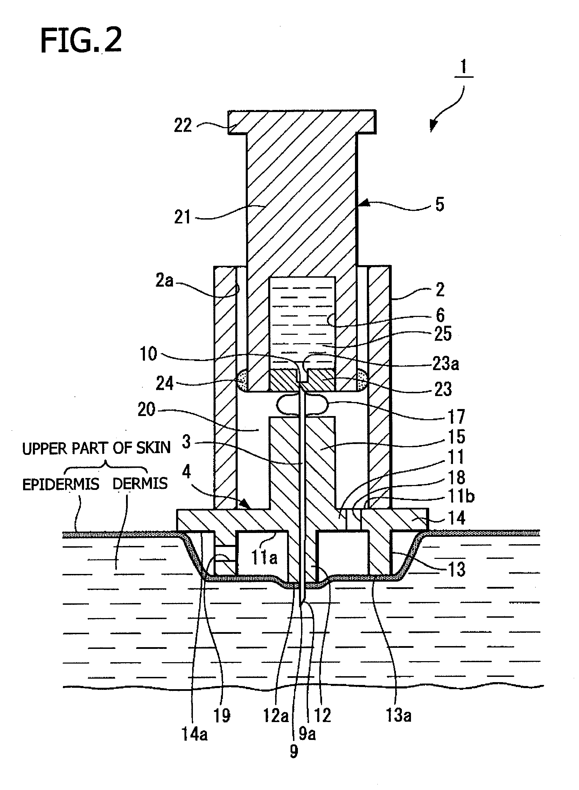

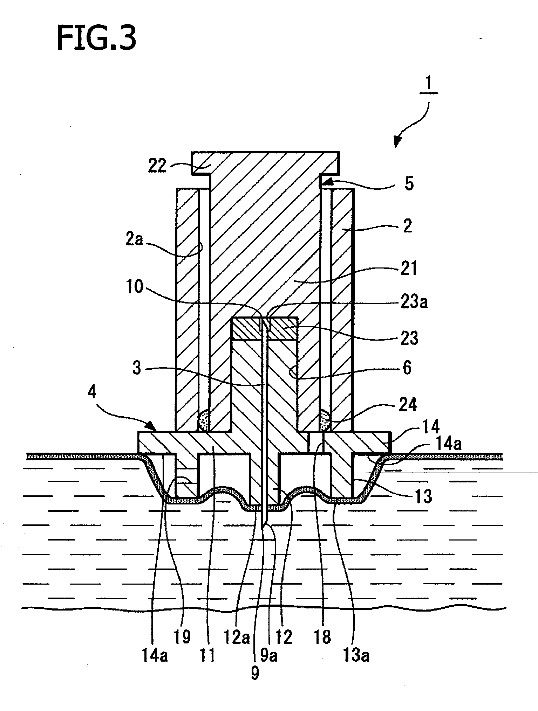

First, a prefilled syringe according to a first embodiment of the present invention (hereinafter referred to as "present embodiment") is described with reference to FIGS. 1 to 3. FIG. 1 is a sectional view showing the prefilled syringe of the present embodiment, and FIG. 2 is a sectional view illustrating a state during puncture of the prefilled syringe of the present embodiment. FIG. 3 is a sectional view illustrating a state after the puncture.

As shown in FIG. 1, the prefilled syringe 1 stores a medicinal solution M to be administrated into a living organism in advance therein and administrates the medicinal solution M into an upper part of the skin. It is to be noted that the upper part of the skin signifies the epidermis and the dermis of the skin. This prefilled syringe 1 includes an outer tube 2, a hollow needle tube 3 having a needle hole, a needle hub 4 for holding the needle tube 3, a medicinal solution vessel 5 having a medicinal solution storage section 6, and a cap 7.

Although, in the present embodiment, various vaccines for preventing various infections such as, for example, influenza are applicable as the medicinal solution M to be stored in the prefilled syringe 1 in advance, the medicinal solution M is not limited to the vaccines. It is to be noted that, in addition to the vaccines, for example, carbohydrate injection solutions of glucose and so forth, injection solutions for electrolyte correction of sodium chloride, potassium lactate and so forth, vitamins, antibiotic injection solutions, contrast agents, steroid drugs, proteolytic enzyme inhibitors, fat emulsions, anti-cancer agents, anesthetics, stimulants, narcotics, heparin calcium, antibody drugs and so forth are applicable.

### [Outer Tube]

### First, the outer tube 2 is described.

The outer tube 2 is formed in a cylindrical shape having a cylindrical hole 2a substantially at the center thereof. The outer tube 2 is open at the opposite ends thereof in the axial direction.

As the material for the outer tube 2, various resins such as, for example, polyvinyl chloride, polyethylene, polypropylene, cyclic polyolefins, polystyrene, poly-(4-methylpentene-1), polycarbonates, acrylic resins, acrylonitrile-butadiene-styrene copolymer, polyesters such as polyethylene terephthalate, butadiene-styrene copolymer, and polyamides (for example, nylon 6, nylon 6,6, nylon 6,10 or nylon 12) are applicable. It is preferable to use, among them, such resins as polypropylene, cyclic polyolefins, polyesters and poly-(4-methylpentene-1) in that they are easy to mold. It is to be noted that preferably the material of the outer tube 2 is substantially transparent in order that the visibility of the inside is assured.

### [Needle Tube]

### Next, the needle tube 3 is described.

As the needle tube 3, a needle tube of a size of the 22 to 33 gauge (outer diameter: 0.2 to 0.7 mm) in accordance with the standard (ISO 9626:1991/Amd. 1:2001 (E)) for needle tubes for medical use of the ISO can be used. Incidentally, where the needle tube 3 is used for administration to the upper part of the skin, a needle tube of the 26 gauge to 33 gauge can be used, and preferably, a needle tube of the 30 to 33 gauge can be used.

A first needle tip 9 to puncture the living organism is provided at one end of the needle tube 3, and a second needle tip 10 to puncture a sealing member of the medicinal solution storage section 6 is provided at the other end of the needle tube 3. This needle tube 3 is a double-ended needle. The first needle tip 9 has a blade surface 9a. The length of the blade surface 9a in a direction in which the needle tube 3 extends (the length is hereinafter referred to as "bevel length B") may be equal to or smaller than 1.4 mm (adult) which is a minimum thickness of the upper part of the skin hereinafter described, and may be equal to or greater than approximately 0.5 mm which is the bevel length when a short bevel is formed on a needle tube of the 33 gauge. In other words, the bevel length B is preferably set within the range of 0.5 to 1.4 mm.

More preferably, the bevel length β is equal to or smaller than 0.9 mm (child) which is a minimum thickness of the upper part of the skin. In other words, it is more preferable to set the bevel length B within the range of 0.5 to 0.9 mm. It is to be noted that the "short bevel" indicates a blade surface having an angle of 18 to 25 degrees with respect to the longitudinal direction of the needle, which is used generally in needles for injection.

The second needle tip 10 has a blade surface 10a. Although the length of the blade surface 10a in the direction in which the needle tube 3 extends can be set arbitrarily, it can be set to an equal length to that of the blade surface 9a of the first needle tip 9. The needle tube 3 is held at an intermediate portion thereof by the needle hub 4.

As the material for the needle tube 3, for example, stainless steel, aluminum, aluminum alloy, titanium, titanium alloy and other metals can be used. Further, as the needle tube 3, a straight needle and a tapering needle which has a tapering structure at least at part thereof can be used. The tapering needle may have such a tapering structure at an intermediate portion thereof that the outer diameter thereof on the second needle tip 10 side is greater than the outer diameter thereof on the first needle tip 9 side. It is to be noted that, in this instance, the first needle tip 9 and the second needle tip 10 are different in shape from each other.

The needle tube 3 is held at an intermediate portion thereof by the needle hub 4. The portion of the needle tube 3 exposed from the needle hub 4 on the second needle tip 10 side is covered with a cover member 17. The cover member 17 is formed as a thin sheet. The cover member 17 is punctured by the second needle tip 10 together with the medicinal solution storage section 6 when the second needle tip 10 of the needle tube 3 punctures the medicinal solution storage section 6.

The cover member 17 is a member which can be punctured by the second needle tip 10 of the needle tube 3 without deformation of the second needle tip 10 and is preferably formed from a member which can be punctured by the second needle tip 10 even with week force of approximately 0.5 to 5 N. As the material for the cover member 17, for example, a PP (polypropylene) or PE (polyethylene) film, rubber, and thermoplastic elastomer are applicable.

The cover member 17 is fitted on the second needle tip 10 side of the needle tube 3. The needle tube 3 and the needle hub 4 are subjected to a gamma ray or electron beam process in a state in which they are covered on the second needle tip 10 side thereof with the cover member 17. Consequently, the second needle tip 10 of the needle tube 3 can be kept in a sterilized state until it punctures the medicinal solution storage section 6.

### [Needle Hub]

Next, the needle hub for holding the needle tube is described. The needle hub 4 includes a hub main body 11 of a substantially disk shape, an adjustment portion 12, a stabilization portion 13, a guide portion 14 serving as a pressure indication portion, and a sealing member contacting portion 15. The needle hub 4 is disposed so as to close up an opening on one end side of the outer tube 2 in a state in which it holds the needle tube 3. At this time, the second needle tip 10 of the needle tube penetrating through the needle hub 4 is disposed in an internal space 20 formed by the cylindrical hole 2a of the outer tube 2 and the needle hub 4.

The adjustment portion 12 and the stabilization portion 13 are provided on one end side of the hub main body 11 while the sealing member contacting portion 15 is provided on the other end side of the hub main body 11. As the material for the needle hub 4, though not limited specifically, for example, materials similar to those of the outer tube 2 described hereinabove can be used. Further, the needle hub 4 may be formed integrally with the outer tube 2.

Further, a first vent hole 18 is provided in the hub main body 11 such that it penetrates through the hub main body 11 from one end side to the other end side. The internal space 20 defined by the cylindrical hole 2a of the outer tube 2 and the needle hub 4 and the outside of the internal space 20 are communicated with each other by the first vent hole 18. Further, a filter may be provided in the first vent hole 18 in order to raise the maintenance of a sterilized state of the internal space 20 defined by the cylindrical hole 2a of the outer tube 2 and the needle hub 4.

It is to be noted that, while, in the present embodiment, an example wherein one first vent hole 18 is provided is described, the number of such first vent holes 18 is not limited to this, but a plurality of first vent holes 18 may be formed in the hub main body 11. Further, since the second needle tip 10 side of the needle tube 3 is covered with the cover member 17, even if a vent is provided in a side face portion of the outer tube 2, the sterilized state of the second needle tip 10 side of the needle tube 3 can be maintained.

### Next, the adjustment portion 12 is described.

The adjustment portion 12 is provided at a central portion of a one end face 11a of the hub main body 11 and formed as a convex portion which protrudes in the axial direction of the hub main body 11. The axis of the adjustment portion 12 coincides with the axis of the hub main body 11. The needle tube 3 penetrates through the adjustment portion 12. Further, an end face of the adjustment portion 12 serves as a needle protruding face 12a from which the first needle tip 9 side of the needle tube 3 protrudes.

The needle protruding face 12a is formed as a flat face perpendicular to the axial direction of the needle tube 3. This needle protruding face 12a defines the depth by which the needle tube 3 punctures after it is brought into contact with the surface of the skin when the needle tube 3 punctures the upper part of the skin. In other words, the depth by which the needle tube 3 punctures the upper part of the skin is determined by the length of the needle tube 3 protruding from the needle protruding face 12a (the length is hereinafter referred to as "protruding length L").

The thickness of the upper part of the skin corresponds to the depth from the surface of the skin to the dermis layer and is included in a range of substantially 0.5 to 3.0 mm. Therefore, the protruding length L of the needle tube 3 can be set within the range from 0.5 to 3.0 mm.

Incidentally, although a vaccine is generally administrated to the brachial region, where administration to the upper part of the skin is taken into consideration, it is considered preferable that a vaccine is administrated to a peripheral region of a shoulder in which the skin is thick, particularly to a deltoid region. Therefore, the thickness of the upper part of the skin at the deltoid was measured on 19 children and 31 adults. This measurement was carried out by sonography of the upper part of the skin having a high ultrasonic reflectivity using an ultrasonic measuring instrument (NP60R-UBM, high resolution echoscope for small animals, NEPA GENE CO., LTD.). It is to be noted that, since the measurement values exhibited a logarithmic normal distribution, the range of MEAN ± 2SD was determined by geometric mean.

As a result, the thickness of the upper part of the skin at the deltoid of the children was 0.9 to 1.6 mm. Meanwhile, the thickness of the upper part of the skin at the deltoid of the adults was 1.4 to 2.6 mm at a distal portion, 1.4 to 2.5 mm at a middle portion and 1.5 to 2.5 mm at a proximal portion. From the foregoing, it was confirmed that the thickness of the upper part of the skin at the deltoid of the children was 0.9 mm or more and that of the adults was 1.4 mm or more. Accordingly, in injection to the upper part of the skin at the deltoid, preferably the protruding length L of the needle tube 3 is set within the range of 0.9 to 1.4 mm.

By setting the protruding length L in this manner, it becomes possible to position the blade surface 9a of the first needle tip 9 at the upper part of the skin with certainty. As a result, the needle hole (medicinal solution discharge port) which is open to the blade surface 9a can be positioned at the upper part of the skin at any position in the blade surface 9a. It is to be noted that, even if the medicinal solution discharge port is positioned at the upper part of the skin, if the first needle tip 9 sticks too deeply into the upper part of the skin, then the medicinal solution M seeps under the subcutaneous part from between the side face of the end portion of the first needle tip 9 and the incised skin. Therefore, it is significant that the blade surface 9a is positioned in the upper part of the skin with certainty.

It is to be noted that, where the prefilled syringe 1 is used for administration to the upper part of the skin, in the case of a thicker needle tube than the 26 gauge, it is difficult to make the bevel length B equal to or smaller than 1.0 mm. Accordingly, in order to set the protruding length L of the first needle tip 9 of the needle tube 3 to a length within the preferable range (0.9 to 1.4 mm), it is preferable to use a needle tube thinner than the 26 gauge.

The needle protruding face 12a is formed such that the distance S from a circumferential edge thereof to the circumferential face of the needle tube 3 is equal to or smaller than 1.4 mm and preferably within a range of 0.3 to 1.4 mm. The distance S from the circumferential edge of the needle protruding face 12a to the circumferential face of the needle tube 3 is set taking it into consideration that pressure is applied to a water blister formed by administration of the medicinal solution to the upper part of the skin. In particular, the needle protruding face 12a is set to a size which is sufficiently smaller than a water blister formed at the upper part of the skin and does not prevent formation of a water blister. As a result, it can be prevented that the needle protruding face 12a presses the skin around the needle tube 3 and the administrated medicinal solution leaks.

### Next, the stabilization portion 13 is described.

The stabilization portion 13 is provided on the one end face 11a of the hub main body 11. The stabilization portion 13 is formed in a continuous tubular shape on a circumferential portion of the one end face 11a. The first needle tip 9 of the needle tube 3 and the adjustment portion 12 are disposed in the cylindrical hole of the stabilization portion 13. In other words, the stabilization portion 13 is formed as a tube which covers the circumference of the adjustment portion 12 through which the needle tube 3 penetrates.

Further, if the first needle tip 9 of the needle tube 3 punctures a living organism as shown in FIG. 2, then the needle protruding face 12a is brought into contact with the surface of the skin and also with an end face 13a of the stabilization portion 13. At this time, since the end face 13a of the stabilization portion 13 contacts with the skin, the prefilled syringe 1 is stabilized and the needle tube 3 can be kept in a posture substantially perpendicular to the skin.

It is to be noted that, even if the end face 13a of the stabilization portion 13 is positioned on the same plane as the needle protruding face 12a or positioned to the first needle tip 9 side of the needle tube 3 with respect to the needle protruding face 12a, the needle tube 3 can be kept in a posture substantially perpendicular to the skin. It is to be noted that, if swelling of the skin when the stabilization portion 13 is pressed against the skin is taken into consideration, then the distance r in the axial direction between the end face 13a of the stabilization portion 13 and the needle protruding face 12a is preferably set to 1.3 mm or less.

Further, the inner diameter d of the stabilization portion 13 is set to a value equal to or higher than the value of the diameter of a water blister formed on the skin. In particular, the inner diameter d of the stabilization portion 13 is set such that the distance T from an inner wall face of the stabilization portion 13 to a circumferential edge of the needle protruding face 12a falls within the range of 4 mm to 15 mm. By this setting, obstruction of formation of a water blister by application of a pressure to a water blister from the inner wall face of the stabilization portion 13 can be prevented.

The distance T from an inner wall face of the stabilization portion 13 to a circumferential edge of the needle protruding face 12a does not specifically have an upper limit only if it is equal to or greater than 4 mm. However, if the distance T is increased, then since the outer diameter of the stabilization portion 13 increases, in the case where the needle tube 3 punctures a thin arm as in the case of a child, it is difficult to contact the entire end face 13a of the stabilization portion 13 with the skin. Therefore, preferably the distance T is defined such that the maximum value thereof is 15 mm taking the thinness of an arm of a child into consideration.

Meanwhile, if the distance S from a circumferential edge of the needle protruding face 12a to a circumferential face of the needle tube 3 is equal to or greater than 0.3 mm, then the adjustment portion 12 does not advance into the skin. Accordingly, if the distance T (equal to or greater than 4 mm) from an inner wall face of the stabilization portion 13 to a circumferential edge of the needle protruding face 12a and the diameter (approximately 0.3 mm) of the needle protruding face 12a are taken into consideration, then the inner diameter d of the stabilization portion 13 can be set to equal to or more than 9 mm.

Further, a second vent hole 19 is formed in the stabilization portion 13 such that it extends from an outer circumferential face to an inner circumferential face of the stabilization portion 13 therethrough. By providing the second vent hole 19 in the stabilization portion 13, when the stabilization portion 13 is brought into contact with the skin as shown in FIG. 2, the space defined by the stabilization portion 13 and the skin and the space on the outer side of the stabilization portion 13 can be communicated with each other. Thus, the first vent hole 18 and the second vent hole 19 configure air communicating means for opening the internal space 20 to the outside.

It is to be noted that the shape of the stabilization portion 13 is not limited to a cylindrical shape, but the stabilization portion 13 may be formed in a prism such as, for example, a square pole or a hexagonal pole having a cylindrical hole at the center thereof.

Next, the guide portion 14 which acts as a pressure indication portion is described.

The guide portion 14 is provided on a side face portion of the hub main body 11. The guide portion 14 is formed as a ring-shaped flange protruding in a radially outward direction of the hub main body 11 from the side face portion of the hub main body 11. The guide portion 14 protrudes substantially perpendicularly to the outer circumferential face of the stabilization portion 13.

Further, the guide portion 14 has a contact face 14a for contacting with the skin. The contact face 14a is a flat face extending substantially in parallel to the end face 13a of the stabilization portion 13. By pressing the stabilization portion 13 until the contact face 14a of the guide portion 14 is brought into contact with the skin, the force of the stabilization portion 13 and the needle tube 3 pressing the skin can always be assured with a value equal to or higher than a predetermined value. Consequently, a portion of the needle tube 3 which protrudes from the needle protruding face 12a (corresponding to the protruding length L) punctures the skin with certainty.

The distance from the contact face 14a of the guide portion 14 to the end face 13a of the stabilization portion 13 is set such that the needle tube 3 can puncture the skin with suitable pressing force applied to the skin from the stabilization portion 13 and the needle tube 3 (refer to FIG. 3). In the following description, this length is referred to as "guide portion height y."

It is to be noted that appropriate pressing force of the needle tube 3 and the stabilization portion 13 is, for example, 3 to 20 N. As a result, the pressing force to the skin from the needle tube 3 and the stabilization portion 13 can be guided to the user by the guide portion 14 and the first needle tip 9 and the blade surface 9a of the needle tube 3 can be positioned with certainty at the upper part of the skin. Therefore, an effect that the sense of security can be provided to the user is achieved.

In particular, in the case where the inner diameter d of the stabilization portion 13 is within a range from 11 to 14 mm, the guide portion height y is set suitably based on the length x (hereinafter referred to as guide portion length x) from a protruding end face of the guide portion 14 to an outer circumferential face of the stabilization portion 13. For example, in the case where the inner diameter d of the stabilization portion 13 is 12 mm, the guide portion height y is set within a range from 2.3 to 6.6 mm, for example, when the guide portion length x is 3.0 mm.

Next, the sealing member contacting portion 15 is described. The sealing member contacting portion 15 is provided at a central portion of the other end face 1 1 b opposing to the one end face 1 1 a of the hub main body 11. This sealing member contacting portion 15 is configured as a projection which protrudes in a substantially column shape in the axial direction of the hub main body 11. The needle tube 3 penetrates through the sealing member contacting portion 15, and the second needle tip 10 of the needle tube 3 protrudes from the end face of the sealing member contacting portion 15.

In the present embodiment, the axis of the needle tube 3 and the axes of the adjustment portion 12 and the sealing member contacting portion 15 coincide with each other. However, even if the axis of the needle tube 3 and the axis of the sealing member contacting portion 15 do not coincide with each other, the intended object can be achieved.

This sealing member contacting portion 15 is disposed in the internal space 20 defined by the cylindrical hole 2a of the outer tube 2 and the needle hub 4 when the opening on one end side of the outer tube 2 is closed up with the needle hub 4.

### [Medicinal Solution Vessel]

Next, the medicinal solution vessel 5 is described.

The medicinal solution vessel 5 has a vessel main body 21 of a substantially cylindrical shape, a pushing portion 22 for being pushed by a user, and the medicinal solution storage section 6. This medicinal solution vessel 5 is inserted in the cylindrical hole 2a of the outer tube 2. Further, one end side of the vessel main body 21 is formed as the pushing portion 22, and the other end side of the vessel main body 21 is formed as the medicinal solution storage section 6. The diameter of the vessel main body 21 is formed smaller than the diameter of the cylindrical hole 2a of the outer tube 2.

The pushing portion 22 is provided on one end side of the vessel main body 21 in the axial direction. The pushing portion 22 is formed as a flange swollen in a radially outward direction of the vessel main body 21. If the pushing portion 22 is pushed by the user, then the vessel main body 21 moves in the cylindrical hole 2a of the outer tube 2 along the axial direction of the cylindrical hole 2a. In other words, in the present embodiment, the medicinal solution vessel 5 plays a role also of a pusher for pushing out the medicinal solution M accommodated in the medicinal solution storage section 6.

### [Medicinal Solution Storage Section]

Next, the medicinal solution storage section 6 is described.

The medicinal solution storage section 6 is provided on the other end side of the vessel main body 21 in the axial direction. This medicinal solution storage section 6 is a recessed portion which is recessed in a substantially cylindrical shape from the other end face of the vessel main body 21 in the axial direction. The medicinal solution M is accommodated in the medicinal solution storage section 6. The diameter of the opening of the medicinal solution storage section 6 is set substantially equal to or a little greater than the diameter of the sealing member contacting portion 15.

Further, a sealing member 23 is attached to the medicinal solution storage section 6 such that it closes up the opening of the medicinal solution storage section 6. Therefore, the medicinal solution M is sealed in a medicinal solution space 25 defined by the medicinal solution storage section 6 and the sealing member 23. The medicinal solution space 25 is an enclosed space sealed airtight by the sealing member 23 such that a sterilized state is maintained. Further, as shown in FIG. 3, the sealing member 23 is pushed, upon using by the sealing member contacting portion 15 of the needle hub 4 to slidably move in the axial direction in the medicinal solution storage section 6. The sealing member 23 is punctured by the second needle tip 10 of the needle tube 3 together with the cover member 17.

Further, at a substantially central portion of one face of the sealing member 23 opposing to the deep end face of the medicinal solution storage section 6, a needle accommodating recessed portion 23a continuing to a pierced hole pierced by the second needle tip 10 is formed. A section of the needle accommodating recessed portion 23a in a direction perpendicular to the axial direction of the sealing member 23 is formed greater than the outer diameter of the needle tube 3. In a state in which administration of the medicinal solution is completed, the second needle tip 10 side of the needle tube 3 is disposed in the needle accommodating recessed portion 23a.

Further, the other medicinal solution M accommodated in a medicinal solution storage section 6 can be exchanged by exchanging the vessel main body 21.

It is to be noted that, although the amount of the medicinal solution M accommodated in the medicinal solution space 25 (volume of the medicinal solution space 25) is not specifically limited, for example, it is preferable to be approximately 0.02 to 2.0 mL and more preferable to be approximately 0.05 to 0.8 mL. In other words, the prefilled syringe 1 is suitable particularly where such a small amount of the medicinal solution is to be administrated.

Further, although the material of the sealing member 23 is not specifically limited, the sealing member 23 is preferably configured from an elastic material in order to assure a good liquid-tightness with the medicinal solution storage section 6. For example, elastic materials such as various rubber materials of natural rubber, butyl rubber, isoprene rubber, butadiene rubber, styrene-butadiene rubber, silicone rubber, isobutylene rubber and so forth, various thermoplastic elastomers of polyurethane-based, polyester-based, polyamide-based, olefin-based and styrene-based type elastomer and the like, or mixtures of them and so forth can be used.

It is to be noted that the sealing member 23 may be formed such that at least an outer circumferential portion thereof is configured from an elastic material as described above and may have a configuration wherein it has a core portion (not shown) configured, for example, from a resin material and the elastic material described above is disposed in such a manner as to cover an outer periphery of the core portion.

Although the material of the medicinal solution vessel 5 having such the configuration as described above is not limited specifically, for example, materials similar to those of the outer tube 2 described hereinabove can be used. It is to be noted that preferably the material of the medicinal solution vessel 5 is substantially transparent in order that the visibility of the inside can be assured. Further, graduations may be formed on the outer circumferential face of the medicinal solution storage section 6 of the medicinal solution vessel 5. Based on the graduations, the amount of the medicinal solution M accommodated in the medicinal solution storage section 6 can be grasped.

Further, a seal member 24 is attached to an outer circumferential face of the vessel main body 21 on the medicinal solution storage section 6 side. The seal member 24 is an O-ring and is provided continuously along a circumferential direction on the outer circumferential face of the vessel main body 21. The seal member 24 and the vessel main body 21 may be formed by two-color molding.

When the medicinal solution vessel 5 is inserted into the cylindrical hole 2a of the outer tube 2, the seal member 24 closely contacts with an outer circumferential face of the vessel main body 21 and an inner wall of the cylindrical hole 2a. Therefore, the cylindrical hole 2a of the outer tube 2 and the vessel main body 21 are sealed fully therebetween. As a result, in an unused state, the internal space 20 defined by the cylindrical hole 2a of the outer tube 2 and the hub main body 11 of the needle hub 4 is sealed airtightly by the seal member 24. Further, since the seal member 24 closely contacts with the outer circumferential face of the vessel main body 21 and the inner wall of the cylindrical hole 2a, the vessel main body 21 can be held stably at an intermediate portion of the cylindrical hole 2a of the outer tube 2.

Although the material of the seal member 24 is not specifically limited, materials similar to those of the sealing member 23 can be used.

### [Cap]

Next, the cap 7 is described.

The cap 7 is a member for sealing the space which surrounds the first needle tip 9 of the needle tube 3 and the adjustment portion 12 and the stabilization portion 13 of the needle hub 4. The cap 7 is removably mounted, in an unused state, on the stabilization portion 13 of the needle hub 4.

This cap 7 is formed as a bottomed tube having a bottom portion on the tip side, in the present embodiment, as a bottomed cylinder. In the unused state, an inside face of the cap 7 is mounted on the outer circumferential face of the stabilization portion 13 to enclose the second vent hole 19 and the inside of the cap 7. By the structure, the sterilized state of the internal space 20 and the inside of the cap 7 is maintained.

When the prefilled syringe 1 is to be used, the cap 7 is removed from the needle hub 4 and the sealing of the first needle tip 9 of the needle tube 3 is canceled. Further, the second vent hole 19 of the needle hub 4 is opened. Consequently, in a state in which the stabilization portion 13 contacts with the skin as illustrated in FIG. 2, the space defined by the stabilization portion 13 and the skin and the internal space 20 can be opened to the outside by the first vent hole 18 and the second vent hole 19.

Although the material of the cap 7 is not limited specifically, for example, materials similar to those of the outer tube 2 or those of the medicinal solution vessel 5 described hereinabove may be used.

### 1-2. Assembling Method of the Prefilled Syringe

Next, an assembling method of the prefilled syringe 1 having the configuration described above is described.

First, the needle tube 3 is fixed to the needle hub 4. At this time, the first needle tip 9 of the needle tube 3 protrudes from the adjustment portion 12 side of the needle hub 4, and the second needle tip 10 protrudes from the sealing member contacting portion 15 of the needle hub 4. Then, the cover member 17 is fitted out on the second needle tip 10 side of the needle hub 4 so as to ensphere it.

Here, the cover member 17 may be placed in a state in which the needle hub 4 is fixed to the outer tube 2, or the needle hub 4 which holds the needle tube 3 in a state in which the cover member 17 is fitted may be fixed to the outer tube 2 such that it closes up the opening of the outer tube 2 on the one end side. Further, the needle hub 4 and the outer tube 2 may be formed integrally with each other as described hereinabove.

Then, the cap 7 is mounted on the stabilization portion 13 side of the needle hub 4. As a result, the second vent hole 19 and the inside of the cap 7 are enclosed. Consequently, the sterilized state of the internal space 20 and the inside of the cap 7 is maintained. It is to be noted that the step of mounting the cap 7 may be carried out before the medicinal solution vessel 5 is inserted into the outer tube 2 after the cover member 17 is fitted on the second needle tip 10 or may be carried out before the cover member 17 is fitted on the second needle tip 10 side.

Then, a sterilization process is carried out for the cap 7, needle hub 4 and needle tube 3 on which the cover member 17 is fitted. As the sterilization process, for example, radiation sterilization by irradiation of a gamma ray or an electron beam or the like can be used.

Then, the medicinal solution vessel 5 wherein the medicinal solution M is filled in the medicinal solution storage section 6 in advance is inserted from the opening of the other end side of the outer tube 2 in an isolator under a sterilized environment. It is to be noted that a sterilization process has already been applied to the medicinal solution vessel 5 and the medicinal solution storage section 6. Also the medicinal solution M is filled in the medicinal solution storage section 6 in a state in which a sterilization process (for example, filter sterilization or the like) has been carried out already under a sterilized environment. At this time, since the second needle tip 10 of the needle tube 3 is covered with the cover member 17, even when the medicinal solution vessel 5 is inserted, the sterilized state can be maintained. The assembly of the prefilled syringe 1 is completed therewith.

By covering the second needle tip 10 side of the needle tube 3 with the cover member 17 immediately after the sterilization process is carried out in this manner, fine particles can be prevented from sticking to the second needle tip 10 during the assembling operation. As a result, the sterilized state of the second needle tip 10 can be kept more safely during the assembly operation.

### 1-3. Method of Use of the Prefilled Syringe

Next, a method of use of the prefilled syringe 1 of the present embodiment is described with reference to FIGS. 1 to 3.

First, the cap 7 is removed from the stabilization portion 13 of the needle hub 4 as illustrated in FIG. 1. Consequently, the sealing of the first needle tip 9 of the needle tube 3 is canceled and the second vent hole 19 of the needle hub 4 is opened. Then, the end face 13a of the stabilization portion 13 is opposed to the skin. Consequently, the first needle tip 9 of the needle tube 3 is opposed to the skin to puncture. Then, as illustrated in FIG. 2, the prefilled syringe 1 is moved substantially perpendicularly to the skin so that the needle tube 3 punctures the skin and the end face 13a of the stabilization portion 13 is pressed against the skin.

In this instance, the needle protruding face 12a of the adjustment portion 12 and the end face 13a of the stabilization portion 13 are positioned on the same plane. Consequently, the needle protruding face 12a of the adjustment portion 12 is placed into contact with the skin to deform the skin so as to be flat, and the first needle tip 9 of the needle tube 3 can puncture the skin by the protruding length L.

Then, the stabilization portion 13 is pressed until the contact face 14a of the guide portion 14 is brought into contact with the skin. Here, the guide portion height y is set such that the needle tube 3 and the stabilization portion 13 can puncture the skin with appropriate pressing force. Therefore, the force of pressing the skin by the stabilization portion 13 becomes a predetermined value. Accordingly, the pressing force of the stabilization portion 13 can be guided to the user and the stabilization portion 13 can be pressed against the skin with appropriate pressing force, and the first needle tip 9 and the blade surface 9a of the needle tube 3 can be positioned with certainty in the upper part of the skin.

Since the guide portion 14 becomes an indicator for guiding the pressing force of the stabilization portion 13 in this manner, the first needle tip 9 of the needle tube 3 can be positioned in the upper part of the skin with certainty. Thus, the medicinal solution can be administrated with certainty into the upper part of the skin and the sense of security of the user can be enhanced.

Further, as the stabilization portion 13 contacts with the skin, the needle tube 3 can be stabilized so that the needle tube 3 straightforwardly punctures the skin. Therefore, deflection which may occurs with the needle tube 3 can be prevented and stabilized administration of the medicinal solution can be achieved.

Furthermore, in the case of a needle having a very small protrusion length, for example, of approximately 0.5 mm, even if the first needle tip 9 is contacted with the skin, it may not sometimes pierce into the skin. However, if the stabilization portion 13 is pressed against the skin to push down the skin in the vertical direction, then a state in which the skin on the inner side of the stabilization portion 13 is pulled and tension is applied to the skin is exerted. Therefore, since the skin becomes less likely to escape with respect to the first needle tip 9 of the needle tube 3, the stabilization portion 13 has an effect also that the first needle tip 9 becomes easier to pierce into the skin.

Further, since the protruding length L is set within the range of 0.5 to 3.0 mm, the first needle tip 9 and the blade surface 9a of the needle tube 3 are positioned in the upper part of the skin with certainty. The adjustment portion 12 closely contacts with and is fixed to a circumference of the needle tube 3 such that a gap does not appear between a portion of the needle tube 3 which penetrates through the adjustment portion 12 and the adjustment portion 12.

Therefore, if the needle protruding face 12a of the adjustment portion 12 is brought into contact with the skin, then the skin around the needle tube 3 can be deformed into a flat state. As a result, the needle tube 3 can puncture the skin by the protruding length L, and the first needle tip 9 of the needle tube 3 can be positioned in the upper part of the skin with certainty.

Then, the user would press the pushing portion 22 to slidably move the vessel main body 21 and the medicinal solution storage section 6, in which the medicinal solution M is filled, in the cylindrical hole 2a along the axial direction of the outer tube 2. Then, the sealing member contacting portion 15 of the needle hub 4 is brought into contact with the sealing member 23. At this time, the second needle tip 10 of the needle tube 3 punctures the cover member 17 and the sealing member 23. It is to be noted that, in FIG. 2, the cover member 17 is interposed between the sealing member 23 and the sealing member contacting portion 15.

Further, the second needle tip 10 is accommodated in the needle accommodating recessed portion 23a and does not protrude from the surface of the sealing member 23 to the deep end face side of the medicinal solution vessel 5. Therefore, the sealing member 23 and the deep end face of the medicinal solution vessel 5 contact with each other without allowing a gap to be produced therebetween.

The communication of the medicinal solution M accommodated in the medicinal solution storage section 6 with the needle tube 3 is completed therewith. Since the second needle tip 10 of the needle tube 3 is covered with the cover member 17 in this manner, the sterilized state of the second needle tip 10 can be maintained until the second needle tip 10 punctures the medicinal solution storage section 6.

Further, the first vent hole 18 and the second vent hole 19 are provided in the needle hub 4. When the pushing portion 22 is operated to move, the air in the internal space 20 is discharged to the outside through the first vent hole 18 and the second vent hole 19. Therefore, the pressure in the space defined by the internal space 20 and stabilization portion 13 and the skin can be prevented from rising. Consequently, the moving operation of the medicinal solution vessel 5 can be carried out readily and smoothly. Further, after administration of the medicinal solution M ends, even if the hand is removed from the pushing portion 22, the medicinal solution vessel 5 can be prevented from moving to the other end portion side of the outer tube 2.

Then, if the pushing portion 22 is pushed toward the needle hub 4 side of the outer tube 2 as shown in FIG. 3, then the sealing member 23 is pushed into the medicinal solution storage section 6 by the sealing member contacting portion 15. Then, since the sealing member 23 slidably moves in the medicinal solution storage section 6 along the axial direction of the medicinal solution storage section 6, the medicinal solution M in the medicinal solution storage section 6 is discharged from the first needle tip 9 into a living organism via the second needle tip 10 of the needle tube 3.

Here, since the needle protruding face 12a of the adjustment portion 12 and the inner diameter d of the stabilization portion 13 are set to appropriate sizes, the filled medicinal solution can be prevented from leaking to the outside of the living organism and can be administrated into the upper part of the skin with certainty. The administration of the medicinal solution M using the prefilled syringe 1 of the present embodiment is completed therewith.

### <2. Second Embodiment>

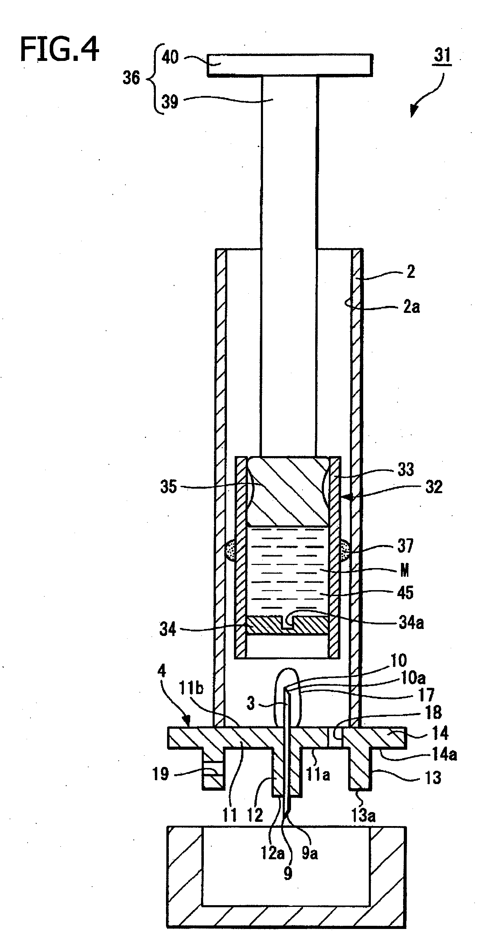

Next, a second embodiment of the prefilled syringe of the present invention is described with reference to FIGS. 4 to 6.

FIGS. 4 to 6 are sectional views showing the prefilled syringe according to the second embodiment.

The prefilled syringe 31 according to the present second embodiment is different from the prefilled syringe 1 according to the first embodiment in that the medicinal solution storage section and the pusher are configured as separate members and the sealing member contacting portion 15 is eliminated from the needle hub 4. Therefore, the medicinal solution storage section and the pusher are described here, and like parts to those of the prefilled syringe 1 are denoted by like reference letters or numerals and overlapping description of them is omitted.

As shown in FIG. 4, the medicinal solution storage section 32 is configured from a tubular member 33 having a substantially cylindrical shape, a first sealing member 34, and a second sealing member 35. The medicinal solution storage section 32 can be exchanged by a medicinal solution storage section in which medicinal solution of the same type or of a different type is accommodated. The tubular member 33 is open at the opposite ends thereof in the axial direction. This tubular member 33 is inserted into the cylindrical hole 2a from the opening on the other end side of the outer tube 2. Then, the tubular member 33 slidably moves in the cylindrical hole 2a in the axial direction of the cylindrical hole 2a by being pushed out by a pusher 36 hereinafter described.

The first sealing member 34 is disposed such that it closes up the opening on the one end side of the tubular member 33 in the axial direction. This first sealing member 34 is opposed to the second needle tip 10 of the needle tube 3 held in the needle hub 4 when the tubular member 33 is inserted into the cylindrical hole 2a of the outer tube 2. Meanwhile, the second sealing member 35 is disposed so as to close up the opening at the other end side of the tubular member 33 in the axial direction.

Further, at a substantially central portion of one face of the first sealing member 34 facing to the second sealing member 35, a needle accommodating recessed portion 34a continuing to the pierced hole into which the second needle tip 10 is to be pierced is formed. The cross section of the needle accommodating recessed portion 34a in a direction perpendicular to the axial direction of the first sealing member 34 is formed greater than the outer diameter of the needle tube 3. In a state in which administration of the medicinal solution is completed, the second needle tip 10 of the needle tube 3 is disposed in the needle accommodating recessed portion 34a.

Further, the medicinal solution M is filled in a medicinal solution space 45 defined by the tubular member 33, a first sealing member 34 and a second sealing member 35. Further, a seal member 37 is provided on an outer circumferential face of the tubular member 33. This seal member 37 is interposed between the outer circumferential face of the tubular member 33 and the cylindrical hole 2a when the tubular member 33 is inserted into the cylindrical hole 2a of the outer tube 2.

The internal space 20 defined by the cylindrical hole 2a and the hub main body 11of the needle hub 4 is sealed airtight by the seal member 37. Further, since the seal member 37 closely contacts with the outer circumferential face of the tubular member 33 and the inner wall of the cylindrical hole 2a, the tubular member 33 can be kept stably at an intermediate portion of the cylindrical hole 2a of the outer tube 2.

Next, the pusher 36 is described.

The pusher 36 has a plunger 39 formed from a bar-like member, and a pushing portion 40 of a substantially disk-like shape. The plunger 39 contacts at one end portion in the axial direction thereof with the second sealing member 35 of the medicinal solution storage section 32. The pushing portion 40 is provided at the other end of the plunger 39 in the axial direction.

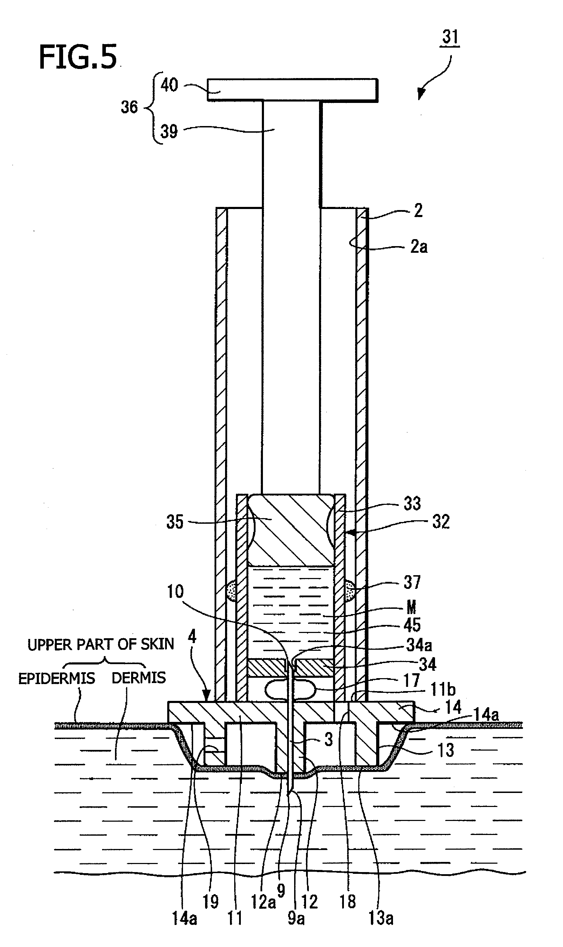

Further, as shown in FIG. 5, if the user pushes the pushing portion 40, then the medicinal solution storage section 32 is pushed out in the axial direction of the cylindrical hole 2a of the outer tube 2 by the plunger 39. Then, the tubular member 33 of the medicinal solution storage section 32 contacts at one end thereof with the other end face 11b of the hub main body 11. At this time, the cover member 17 is punctured by the second needle tip 10 of the needle tube 3 together with the first sealing member 34 of the medicinal solution storage section 32. Consequently, liquid communication to the needle tube 3 is carried out.

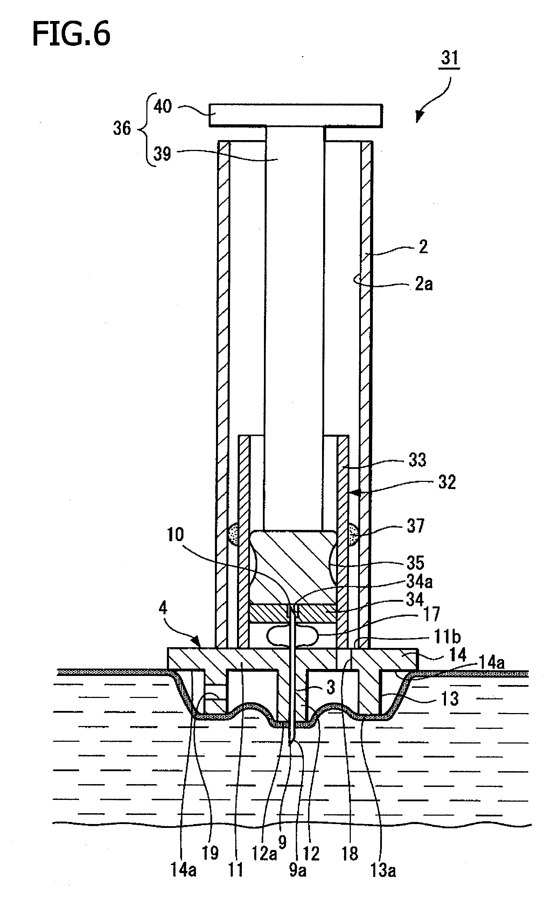

Further, if the pushing portion 40 is further pushed after the second needle tip 10 punctures the medicinal solution storage section 32 and then the liquid communication into the needle tube 3 is completed, then the second sealing member 35 is pushed out to the first sealing member 34 side by the plunger 39 as illustrated in FIG. 6. By slidably moving the second sealing member 35 in the axial direction in the tubular member 33, the medicinal solution M in the medicinal solution storage section 32 is discharged into a living organism from the first needle tip 9 through the second needle tip 10 of the needle tube 3.

Since the configuration of the other part of the prefilled syringe 31 is similar to that of the prefilled syringe 1 according to the first embodiment described hereinabove, description of the same is omitted herein. Also by the prefilled syringe 31 having such a configuration as described above, operation and effects similar to those achieved by the prefilled syringe 1 according to the first embodiment described hereinabove can be achieved.

It is to be noted that the present invention is not limited to the embodiments described hereinabove and shown in the drawings but can be carried out in various modified forms without departing from the scope of the present invention described in the claims. It is to be noted that, while, in the embodiments described above, an example wherein a pressure indication portion is formed as a flange, the pressure indication portion is not limited to this. For example, the pressure indication portion may be formed by cutting away an outer circumferential face of a cylindrical portion or a stabilization portion substantially vertically to provide a stepped portion.

Further, while an example wherein an adjustment portion, a stabilization portion and a guide portion serving as a pressure indication portion are provided on a needle hub in order to administrate the medicinal solution to the upper part of the skin is described above, the structure is not limited to this, and the adjustment portion, stabilization portion and guide portion need not be provided. Further, the prefilled syringe of the present invention can be applied also to a prefilled syringe which feeds a medicinal solution to a location deeper than the upper part of the skin. Further, also the size of the needle tube is not limited to that of the 22 gauge to 33 gauge but is suitably selected in accordance with an object.

### EXPLANATIONS OF LETTERS OR NUMERALS

1, 31: Prefilled syringe, 2: Outer tube, 2a: Cylindrical hole, 3: Needle tube (Double-ended needle), 4: Needle hub, 5: Medicinal solution vessel (Pusher), 6, 32: Medicinal solution storage section, 9: First needle tip, 10: Second needle tip, 11: Hub main body, 12: Adjustment portion, 13: Stabilization portion, 14: Guide portion, 15: Sealing member contacting portion, 17: Cover member, 18: First vent hole, 19: Second vent hole, 20: Internal space, 21: Vessel main body, 22, 40: Pushing portion, 23: Sealing member, 24, 37: Seal member, 25, 45: Medicinal solution space, 33: Tubular member, 34: First sealing member, 36: Pusher, 35: Second sealing member, 39: Plunger, B: Bevel length, L: Protruding length, S: Distance from a circumferential edge of the needle protruding face to a circumferential face of the needle tube, T: Distance from an inner wall face of the stabilization portion to an outer circumferential face of the adjustment portion, x: Guide portion length, y: Guide portion height, d: Inner diameter

## Claims

1. A prefilled syringe, comprising:
a double-ended needle having a first needle tip provided at one end thereof for puncturing a living organism and a second needle tip provided at the other end thereof for being injected with a medicinal solution;
an outer tube having a cylindrical hole into which the second needle tip of the double-ended needle is disposed and which is open at the opposite ends thereof;
a needle hub configured to hold an intermediate portion of the double-ended needle and close up the opening on the one end side of the outer tube;
a medicinal solution storage section movably inserted in the outer tube and filled with the medicinal solution by a sealing member which seals the one end;
a movable pusher configured to operate the medicinal solution storage section for movement in an axial direction of the outer tube; and
a cover member configured to cover the second needle tip side of the double-ended needle exposed from the needle hub.

2. The prefilled syringe according to claim 1, wherein the cover member is punctured by the second needle tip of the double-ended needle when the medicinal solution is to be injected.

3. The prefilled syringe according to claim 1, wherein the cover member is formed from a member which can be punctured by the second needle tip of the double-ended needle without causing deformation of the second needle tip.

4. The prefilled syringe according to claim 1, wherein the medicinal solution storage section is configured for replacement.

5. The prefilled syringe according to claim 1, wherein an adjustment section having a needle protruding face through which the first needle tip of the double-ended needle protrudes is provided on a circumference of the double-ended needle.

6. The prefilled syringe according to claim 1, wherein a stabilization section disposed so as to cover a circumference of the first needle tip of the double-ended needle and having an end face which contacts with a skin when the double-ended needle punctures a living organism is provided.

7. An assembling method of a prefilled syringe, comprising:
a step of fixing a double-ended needle, which has a first needle tip provided at one end thereof and configured to puncture a living organism and a second needle tip having medicinal solution injected therein and disposed in a cylindrical hole of an outer tube, to a needle hub and covering the second needle tip side of the double-ended needle held by the needle hub with a cover member;
a step of applying a sterilization process to the double-ended needle, whose second needle tip side is covered with the cover member, and the needle hub; and
a step of inserting, into the cylindrical hole of the outer tube whose opening on the one end side is closed up with the needle hub, the medicinal solution storage section in which the medicinal solution is filled and a movable pusher provided so as to operate the medicinal solution storage section for movement in an axial direction of the cylindrical hole from the other end side of the outer tube under a sterilized environment.
